# DEMANDE DE BREVET EUROPEEN

(11) **EP 3 854 368 A1**
(43) Date de publication de la demande: **28.07.2021**
(21) Numéro de dépôt: 21150030.1
(22) Date de dépôt: 04.01.2021
(51) Int. Cl.: A61G 12/00, A61M 16/01

(54) **CHARIOT HOSPITALIER DE TRANSPORT DE BOUTEILLE DE GAZ AVEC COMPARTIMENT DE RANGEMENT DU TUYAU FLEXIBLE**

(30) Priorité: 23.01.2020 FR 2000627
(71) Demandeur: L'AIR LIQUIDE, SOCIETE ANONYME POUR L'ETUDE ET L'EXPLOITATION DES PROCEDES GEORGES CLAUDE, 75007 Paris (FR); Air Liquide Santé France, 75007 Paris (FR)
(72) Inventeur: THEVENET, Louise, 94250 Gentilly (FR); QUENEDEY, Jean-Patrice, 78350 Jouy en Josas (FR); CHANCEL, Fabien, 78350 Jouy en Josas (FR)
(74) Mandataire: Air Liquide

(57) **Abrégé**

L'invention concerne un chariot hospitalier (1) de transport de bouteille de gaz mobile comprenant une carcasse (2) délimitant un espace interne (3) et comprenant une paroi de toit (4) surmontant ledit espace interne (3), plusieurs roues (10) pour déplacer le chariot sur le sol et un système de distribution (5) de gaz comprenant une extrémité de raccordement (5a) et à laquelle est raccordée fluidiquement une extrémité amont (6a) d'un conduit flexible (6). La paroi de toit (4) comprend une ouverture supérieure (7) en communication avec l'espace interne (3). La partie intermédiaire (6b) du conduit flexible (6) vient se loger dans l'espace interne (3) au travers de l'ouverture supérieure (7), en particulier dans un compartiment de rangement (11) est à paroi périphérique souple (11a) et a une forme de sac.

## Description

L'invention porte sur un chariot de transport de bouteille(s) de gaz permettant de transporter une ou plusieurs bouteilles de gaz d'un endroit à un autre au sein d'un bâtiment hospitalier par exemple, c'est-à-dire un chariot hospitalier.

En milieu médical, par exemple en cabinet dentaire ou au sein d'une clinique ou d'un hôpital, il est courant d'utiliser des gaz médicaux aux patients, par exemple des gaz analgésiques, tel le N₂O, des gaz d'assistance respiratoire, tel l'oxygène ou l'air médical, ou autres. Ces gaz peuvent être acheminés par un réseau de canalisations ou conditionnés en bouteilles de gaz, comme décrit par EP-A-629812.

Les bouteilles de gaz doivent être transportées par le personnel soignant, tel que médecins, infirmières ou autres, jusqu'à l'endroit où les soins doivent être effectués, par exemple une salle de soins ou une chambre.

Pour ce faire, on utilise un chariot de transport de bouteille qui comprend souvent un plateau portant un ou des appareils, matériels ou instruments médicaux, ou des objets ou autres servant pendant les soins, tels que masques, filtre anti-bactérien, produits désinfectants, papiers, stylos... La (ou les) bouteille est raccordée fluidiquement à un système de distribution de gaz comprenant classiquement un ou plusieurs conduits, un ou des connecteurs etc...

Ainsi, EP-A-2574361 enseigne un chariot de soins mobile qui comprend une carcasse délimitant un logement interne recevant une bouteille de gaz, et à laquelle sont fixés deux bras-porteurs portant une structure plateau surmontant le chariot, sur laquelle peuvent être disposés des instruments et/ou appareils médicaux, ou autres.

D'autres chariots de transport de bouteilles de gaz sont décrits par EP-A-1977712, FR-A-2926728 et FR-A-2910362.

Afin de distribuer le gaz aux patients, on utilise en général un tuyau ou conduit flexible venant se raccorder par, son extrémité amont, au système de distribution de fluide du chariot, par exemple vient un connecteur adapté, alors que son extrémité aval alimente une interface respiratoire servant à fournir le gaz au patient, tel qu'un masque respiratoire ou analogue.

Or, ce tuyau ou conduit flexible pose un problème d'encombrement car il encombre l'espace autour du chariot puisqu'en général il pend le long des parois latérales du chariot ou est enroulé et posé sur le dessus ou sur le plateau le surmontant.

KR 20180115464, US 5144945 et DE 202006017375 proposent des chariots de soins mobiles comprenant des compartiments internes servant au rangement des tuyaux, masques et autres instruments. Toutefois, avec ces types de chariots, le compartiment interne peut être contaminé par des pathogènes ou autres provenant du patient, lors des phases d'interruption de la thérapie, c'est-à-dire lorsque le tuyau et le masque sont rangés temporairement dans le compartiment, et ne permet pas une garantir une évacuation du tuyau et du masque dans de bonnes conditions d'hygiène et de sécurité pour le personnel soignant, après usage.

Le problème est alors de proposer un chariot de transport d'une ou plusieurs bouteilles de gaz amélioré, lequel ne pose pas les problèmes et inconvénient susmentionnés liés au tuyau flexible et du masque qui y est raccordé.

La solution concerne alors un chariot de transport de bouteille(s) de gaz mobile comprenant :
- une carcasse délimitant un espace interne et comprenant une paroi de toit surmontant ledit espace interne,
- plusieurs roues pour déplacer le chariot sur le sol,
- un système de distribution de gaz comprenant une extrémité de raccordement située à l'extérieur de la carcasse et à laquelle est raccordée fluidiquement une extrémité amont d'un conduit flexible,
et dans lequel :
- la paroi de toit comprend une ouverture supérieure en communication avec au moins une partie dudit espace interne, au moins une partie dudit conduit flexible venant se loger dans ladite au moins une partie de l'espace interne au travers de l'ouverture supérieure, et
- un compartiment de rangement est agencé dans l'espace interne de la carcasse en-dessous de l'ouverture supérieure, ladite ouverture supérieure débouchant dans ledit compartiment de rangement de manière à ce qu'au moins une partie intermédiaire du conduit flexible vienne se loger dans ledit compartiment de rangement,
caractérisé en ce que le compartiment de rangement comprend un sac à paroi périphérique souple.

Un compartiment de rangement comprenant un sac à paroi périphérique souple selon l'invention permet non seulement de ranger facilement le tuyau pendant son utilisation mais aussi de garantir une bonne hygiène de l'intérieur du chariot en éviter que les parois internes ne soient en contact direct avec le tuyau, voire avec le masque, donc à éviter qu'elles ne soient contaminées par des pathogènes (virus, bactéries...) ou autres contaminants pouvant se trouver sur le tuyau et/ou le masque et provenant en particulier du patient ayant été traité ou pendant son traitement.

En effet, lorsque de tels pathogènes ou autres contaminants sont présents sur le tuyau et/ou le masque, ceux-ci sont enfermés à l'intérieur du sac à paroi flexible selon l'invention, ce qui empêche leur propagation à l'intérieur du chariot.

De plus, en fin de traitement du patient, le personnel soignant peut simplement laisse tomber (i.e. insérer totalement) le tuyau et la masque au fond du sac puis de le refermer hermétiquement, avant de l'évacuer en toute sécurité sanitaire.

Selon le mode de réalisation considéré, le chariot de l'invention peut comprendre l'une ou plusieurs des caractéristiques suivantes :
- le système de distribution de gaz comprend une extrémité de raccordement située à l'extérieur de la carcasse.
- une portion amont du système de distribution de gaz est agencée dans la carcasse de manière à venir se raccorder fluidiquement à la ou aux bouteilles de gaz.
- le système de distribution de gaz comprend une ou des conduites de gaz, un ou des connecteurs, etc....
- le compartiment de rangement est un sac à paroi souple.
- le compartiment de rangement comprend un sac plastique.
- le sac à paroi souple est muni d'un système ou de moyens de fermeture permettant de fermer, de préférence hermétiquement, le sac après insertion totale du (des) tuyau et/ou du masque dans le volume interne dudit sac.
- le sac à paroi souple comprend des moyens de fixation permettant de le fixer à la carcasse, de préférence à la paroi de toit de la carcasse.
- le sac à paroi souple est agencé dans le volume interne de la carcasse.
- le conduit flexible comprend une extrémité amont, une extrémité aval à raccorder fluidiquement à une interface respiratoire et une partie intermédiaire située entre lesdites extrémité amont et aval.
- l'extrémité amont et l'extrémité aval sont situées hors de la carcasse lorsque la partie intermédiaire du conduit flexible est logée dans le compartiment de rangement, i.e. dans le sac à paroi souple, c'est-à-dire lorsqu'au moins une partie du conduit flexible est rangée dans le compartiment de rangement, i.e. dans le sac à paroi souple.
- un dispositif de guidage est agencé au niveau de l'ouverture supérieure pour guider le conduit flexible lorsqu'il est introduit ou extrait du compartiment de rangement au travers de l'ouverture supérieure, en particulier lorsque le conduit flexible coulisse vers l'intérieur ou l'extérieur dudit compartiment de rangement i.e. dans le sac à paroi souple.
- le dispositif de guidage a une forme générale de diabolo comprenant un passage central logeant la partie intermédiaire du conduit flexible.
- le dispositif de guidage comprend un corps en forme de diabolo comprenant, outre le passage central, des parois supérieure et inférieure formant des flasques parallèles séparées par une gorge annulaire.
- les deux parois supérieure et inférieure du dispositif de guidage viennent prendre en sandwich les surfaces supérieure et inférieure de la paroi de toit, au niveau du rebord périphérique de l'ouverture supérieure.
- le rebord périphérique de l'ouverture supérieure vient se loger dans la gorge annulaire.
- le dispositif de guidage est formé d'un polymère, notamment d'un élastomère.
- un contrepoids coulissant est porté par la partie intermédiaire du conduit flexible.
- le conduit flexible comprend une aval raccordée fluidiquement à une interface respiratoire, en particulier un masque respiratoire ou analogue, par exemple un masque nasal ou facial (i.e. bucco-nasal).
- le conduit flexible comprend une aval raccordée fluidiquement à une interface respiratoire par l'intermédiaire d'au moins un élément de raccordement fluidique, tel un (ou des) connecteur ou analogue.
- la majeure partie du conduit flexible se trouve dans le compartiment de rangement, i.e. dans le sac à paroi souple, lorsque ledit conduit flexible est rangé/logé dans le compartiment de rangement, i.e. dans le sac à paroi souple.
- la partie intermédiaire du conduit flexible se trouvant dans le compartiment de rangement, i.e. dans le sac à paroi souple, forme un coude ou une demi-boucle, typiquement en forme de « U », voire une (des) boucles en forme de « O » selon la longueur du conduit flexible.
- le conduit flexible a une longueur comprise entre 1 et 10 m, de préférence entre 2 et 5 m.
- l'espace interne de la carcasse est dimensionné pour loger en outre une ou plusieurs bouteilles de gaz, typiquement 1 ou 2 bouteilles de gaz.
- la paroi de toit comprend une ouverture supérieure de forme circulaire, ovale, polygonale, par exemple carrée ou rectangulaire, ou toute autre forme.
- la carcasse comprend des parois périphériques latérales, de préférence 4 parois latérales.
- la carcasse comprend une paroi de dessous ou de fond.
- les parois périphériques latérales se projettent verticalement entre la paroi de fond et la paroi de toit, i.e. de dessus.
- l'espace interne est compris à l'intérieur et/ou délimité par lesdites parois périphériques latérales, paroi de toit et paroi de fond de la carcasse.
- les parois périphériques latérales sont solidaires les unes des autres.
- la surface externe de la paroi de toit est conformée extérieurement en un plateau de soins pouvant recevoir des matériels, des instruments, des appareils, des dispositifs ou autres, utilisés pendant les soins d'un patient.
- il comprend 4 roues pour déplacer le chariot sur le sol.
- il comprend une ouverture latérale agencée dans l'une des parois périphériques latérales et donnant accès à l'espace interne.
- il comprend un tiroir basculant configuré et dimensionné pour recevoir au moins une bouteille de gaz, de préférence deux bouteilles de gaz.
- le tiroir basculant est agencé au niveau de (e.g. dans) l'ouverture latérale et pivotant par rapport à la base de l'ouverture latérale de la carcasse.
- la paroi externe du tiroir est dimensionnée pour obturer complètement l'ouverture latérale, c'est-à-dire que ses dimensions sont telles qu'elles recouvrent toute ou quasiment toute la surface de l'ouverture latérale aménagée dans la paroi périphérique de la carcasse.
- le tiroir basculant comprend un fond de tiroir sur lequel repose la ou les bouteilles de gaz.
- selon un autre mode de réalisation, il comprend une porte pivotante ou coulissante agencée au niveau de l'ouverture latérale de la carcasse de manière à obturer complètement l'ouverture latérale, c'est-à-dire que ses dimensions sont telles que la porte recouvre toute ou quasiment toute la surface de l'ouverture latérale aménagée dans la paroi périphérique de la carcasse.
- la paroi externe du tiroir ou de la porte pivotante ou coulissante comprend des moyens de préhension permettant à l'utilisateur de la saisir manuellement pour opérer une ouverture ou une fermeture du tiroir ou de la porte, par exemple une découpe ou une poignée qui est conçue pour pouvoir être saisie à une main par l'utilisateur.
- la ou les bouteilles de gaz contiennent un gaz médical, i.e. gaz ou mélange gazeux.
- la ou les bouteilles de gaz contiennent un gaz ou mélange gazeux choisi parmi l'oxygène, l'air, un mélange N₂O/O₂ ou autre.
- la ou les bouteilles de gaz ont un corps de forme générale cylindrique.
- la ou les bouteilles de gaz sont équipées d'un robinet de distribution de gaz, avec ou sans système de détente de gaz intégré, c'est-à-dire de type robinet simple ou type robinet à détendeur intégré ou RDI.
- la ou les bouteilles de gaz contiennent du gaz sous pression, typiquement conditionné à une pression maximale de l'ordre de 250 à 300 bar abs.
- la ou les bouteilles de gaz sont en métal, tel de l'acier ou un alliage d'aluminium, ou en matériaux composites.
- la carcasse a une forme générale approximativement parallélépipédique rectangle.
- les roues sont fixées à un châssis, ledit châssis portant en outre la carcasse.
- alternativement, les roues sont fixées directement à la carcasse du chariot.
- la carcasse et/ou le tiroir sont en métal ou en polymère, ou les deux.
- la carcasse comprend des moyens de contrôle des déplacements, par exemple un arceau ou des poignées, manipulables par l'utilisateur afin de pouvoir pousser et diriger le chariot durant ses déplacements sur le sol.
- la carcasse est formée d'une seule pièce ou de plusieurs sous-unités fixées les unes aux autres.

Un chariot selon l'invention est particulièrement bien adapté à une utilisation pour transporter une ou deux bouteilles de gaz au sein d'un lieu de soins ou un bâtiment hospitalier, du type hôpital, clinique, cabinet dentaire, cabinet médical ou autre.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un chariot hospitalier selon l'invention ; et
Fig. 2 correspond à la partie encadrée de la Fig. 1 incluant le compartiment de rangement du conduit flexible.

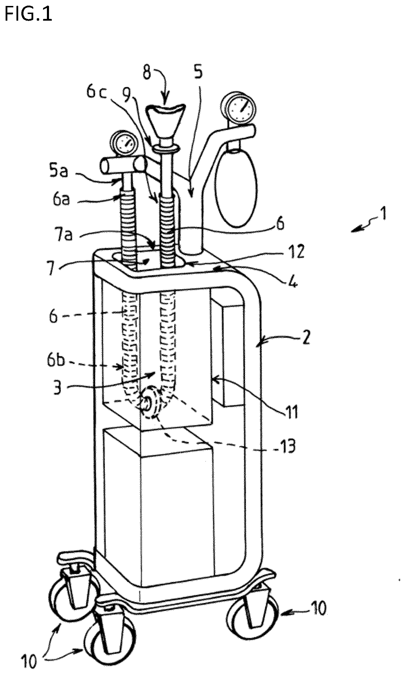

Fig. 1 schématise un mode de réalisation d'un chariot hospitalier 1 de transport de bouteille de gaz selon l'invention.

Ce chariot 1 de transport de bouteille de gaz mobile comprend une carcasse 2 délimitant un espace interne 3. La carcasse 2 comprend par ailleurs une ouverture supérieure 7 agencée dans la paroi de toit 4, et donnant accès à un compartiment de rangement 11 logé dans l'espace interne 3, c'est-à-dire que le compartiment de rangement 11 occupe une partie de l'espace interne 3 de la carcasse 2.

Afin d'assurer ses déplacements sur le sol au sein d'un bâtiment hospitalier par exemple, sont prévues plusieurs roues 10, par exemple 4 roues, agencées sur un châssis portant en outre la carcasse 2 ou, selon un autre mode de réalisation, directement fixées à la carcasse 2, par exemple à la paroi de fond de la carcasse 2.

Ici, la carcasse comprend 4 parois périphériques latérales, une paroi de dessus ou de toit 4 et une paroi de dessous ou de fond (non visible), fixées ou solidaires les unes des autres, et forment ensemble une structure générale en parallélépipède rectangle. Les 4 parois périphériques latérales se projettent verticalement entre la paroi de fond et la paroi de toit 4, et sont fixées à celles-ci de manière à définir entre elles, l'espace interne 3 de la carcasse 2.

La carcasse 2 peut être formée d'une seule pièce ou de plusieurs sous-unités fixées les unes aux autres. Elle peut être réalisée en métal, en polymère ou les deux.

Afin de permettre l'insertion d'une ou plusieurs bouteilles de gaz dans l'espace interne 3 de la carcasse 2, il est prévu une ouverture latérale aménagée dans une des parois périphériques de la carcasse 2, laquelle est fermée par une porte ou un tiroir basculant configuré et dimensionné pour recevoir la ou les bouteilles de gaz, par exemple deux bouteilles agencées en parallèles l'une de l'autre. Chaque bouteille de gaz a une forme générale cylindrique allongée axialement et comprend un robinet ou robinet à détendeur intégré (RDI) servant à contrôler le débit et/ou la pression du gaz délivré. La ou les bouteilles de gaz contiennent avantageusement un gaz médical, par exemple un gaz ou mélange gazeux choisi parmi l'oxygène, l'air, un mélange N₂O/O₂ ou autre.

En outre, il peut aussi être prévu des moyens de contrôle des déplacements du chariot 1, par exemple un arceau ou des poignées, manipulables par l'utilisateur afin de lui permettre de pousser le chariot 1 et de guider ses déplacements sur le sol.

Comme illustré en Fig. 1, le chariot 1 comprend aussi un système de distribution de gaz 5 comprenant une portion aval avec une extrémité de raccordement 5a faisant saillie à l'extérieur de la carcasse 2, par exemple au-dessus de la paroi de toit 4.

Une portion amont du système de distribution de gaz 5 est agencée dans la carcasse 2 de manière à venir se raccorder fluidiquement à la ou aux bouteilles de gaz, en particulier à un robinet ou robinet à détendeur intégré (RDI) équipant chaque bouteille, afin de permettre un soutirage et une distribution ensuite du gaz qu'elles contiennent. Ce système de distribution de gaz 5 comprend classiquement des conduites de gaz, des connecteurs, etc....

La portion aval du système de distribution de gaz 5 qui est terminée par l'extrémité de raccordement 5a, est configurée pour permettre le raccordement fluidique d'un conduit flexible 6, c'est-à-dire un tuyau souple, servant à véhiculer du gaz.

On peut aussi prévoir un système de basculement agencé dans la carcasse 2 permettant de basculer, de préférence automatiquement, d'une bouteille à une autre pour assurer une continuité de l'approvisionnement en gaz lorsqu'une bouteille est (quasi) vide.

Le conduit flexible 6, e.g. un tuyau en polymère, comprend une extrémité amont 6a, une extrémité aval 6c à laquelle vient se raccorder fluidiquement, directement ou via un dispositif de connexion 9, tel un connecteur ou autre, une interface respiratoire 8, tel un masque respiratoire ou analogue, et une partie intermédiaire 6b située entre lesdites extrémité amont 6a et aval 6c.

Selon l'invention, la paroi de toit 4 comprend une ouverture supérieure 7 en communication avec au moins une partie dudit espace interne 3, c'est-à-dire que l'ouverture supérieure 7 débouche dans une partie de l'espace interne 3 et plus particulièrement dans un compartiment de rangement 11 agencée dans ledit espace interne 3 de la carcasse 2.

Cette ouverture supérieure 7 et le compartiment de rangement 11 permettent de recevoir et de ranger au moins la partie intermédiaire 6b du conduit flexible 6 puisque celui-ci vient se loger dans le compartiment de rangement 11 via l'ouverture supérieure 7.

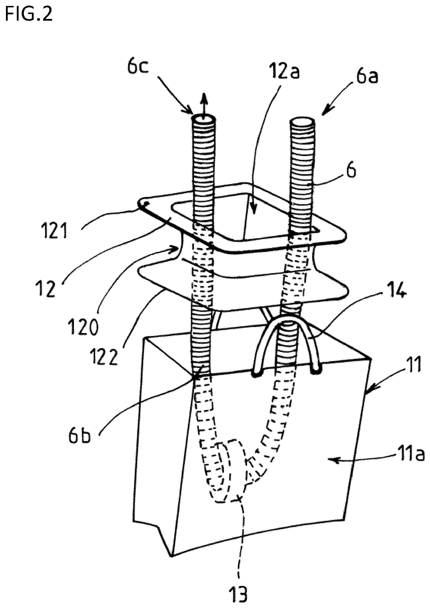

En effet, comme illustré sur les Fig. 1 et Fig. 2, lorsque le conduit flexible 6 n'est pas utilisé, par exemple pendant le stockage du chariot ou pendant ses déplacements sur le sol au sein d'un bâtiment, il convient de ranger le conduit flexible 6 afin d'éviter que celui-ci ne se déroule et tombe sur le sol et/ou ne gêne les déplacements du chariot 1.

Pour ce faire, selon l'invention, le conduit flexible 6 et plus précisément sa partie intermédiaire 6b, i.e. sa portion centrale, est rentrée par l'utilisateur, tel un personnel soignant, e.g. une infirmière ou un médecin, dans le compartiment de rangement 11 i.e. dans un sac à paroi souple, via l'ouverture supérieure 7 aménagée dans la paroi de toit 4. En fait, les extrémités amont 6a et aval 6c (avec le masque 8) du conduit flexible 6 restent préférentiellement à l'extérieur de la carcasse 2.

Afin de faciliter et guider l'insertion et l'extraction par coulissement du conduit flexible 6, i.e. de sa partie intermédiaire 6b, dans le compartiment de rangement 11, i.e. dans le sac à paroi souple, via l'ouverture supérieure 7, on prévoit préférentiellement un dispositif de guidage 12 qui est agencé au niveau de l'ouverture supérieure 7, par exemple fixé au rebord périphérique 7a de l'ouverture supérieure 7 pratiquée dans la paroi de toit 4.

Comme illustré en Fig. 2, le dispositif de guidage 12 a ici un corps ayant une forme générale de diabolo comprenant un passage central 12a logeant la partie intermédiaire 6b du conduit flexible 6, c'est-à-dire que la partie intermédiaire 6b du conduit flexible 6 coulisse dans ce passage central 12a du dispositif de guidage 12, lorsque l'utilisateur introduit ou extrait le conduit flexible 6 du compartiment de rangement 11 i.e. dans le sac à paroi souple.

Plus précisément, le dispositif de guidage 12 comprend un corps en forme de diabolo comprenant deux parois supérieure et inférieure 121, 122 formant des flasques parallèles, i.e. des flasques latérales situées de part et d'autre du corps en forme de diabolo, séparées par une gorge annulaire 120. Ces deux parois supérieure et inférieure 121, 122 viennent prendre en sandwich les surfaces supérieure et inférieure de la paroi de toit 4, au niveau du rebord périphérique 7a de l'ouverture supérieure 7, alors que ledit rebord périphérique 7a de l'ouverture supérieure 7 vient se loger dans la gorge 120.

Préférentiellement, afin de faciliter l'introduction du conduit flexible 6 dans le compartiment de rangement 11 i.e. dans le sac à paroi souple, on peut prévoir un contrepoids coulissant 13 agencé sur la partie intermédiaire 6b du conduit flexible 6.

Dans tous les cas, lorsque le conduit flexible 6 est positionné dans le compartiment de rangement 11, il y forme une (ou des) demi-boucle, ici en forme de U, comme illustré en Fig. 2. Bien entendu, selon la longueur du conduit flexible 6, plusieurs boucles peuvent se former, en forme de « O » et de « U ». Typiquement, le conduit flexible peut avoir une longueur comprise entre 1 et 10 m.

Préférentiellement, le compartiment de rangement 11 comprend un sac flexible à une paroi périphérique souple 11a, par exemple en polymère, i.e. plastique. Avantageusement, le compartiment de rangement 11 est un sac de type poche en plastique, comme visible en Fig. 2 de manière à permettre notamment de garantir une bonne hygiène de l'intérieur du chariot, c'est-à-dire sans risque de le contaminer avec des pathogènes ou autres contaminants pouvant se trouver sur le tuyau ou l'interface respiratoire 8, tel un masque respiratoire, et part ailleurs de faciliter l'évacuation du tuyau flexible et de l'interface respiratoire 8, après usage, là encore, dans de bonnes conditions d'hygiène. Il comprend des moyens de fixation 14, telles des anses ou autres, permettant de le fixer à la carcasse 2, de préférence à la paroi de toit 4 de la carcasse 2 du chariot 1.

Autrement dit, le sac flexible formant le compartiment de rangement 11 est dimensionné pour loger le tuyau souple et le masque.

Préférentiellement, le sac à paroi souple est aussi muni d'un système de fermeture, par exemple un (ou des) lacet, cordon, fil, bande, élément adhésif ou autre, permettant de fermer, de préférence hermétiquement, le sac après insertion totale du tuyau et/ou du masque dans le volume interne du sac.

Le sac flexible à une paroi périphérique souple 11a formant le compartiment de rangement 11 est logé à l'intérieur de la carcasse du 2 chariot 1, comme illustré en Fig. 1.

D'une façon générale, un chariot selon l'invention est adapté à une utilisation pour transporter au moins une bouteille de gaz, de préférence une ou des bouteilles de gaz contenant un gaz ou mélange gazeux médical, typiquement un gaz ou mélange gazeux choisi parmi l'oxygène, l'air et un mélange N₂O/O₂ (tel du MEOPA).

## Revendications

1. Chariot (1) de transport de bouteille de gaz mobile comprenant :
- une carcasse (2) délimitant un espace interne (3) et comprenant une paroi de toit (4) surmontant ledit espace interne (3),
- plusieurs roues (10) pour déplacer le chariot sur le sol et
- un système de distribution (5) de gaz comprenant une extrémité de raccordement (5a) et à laquelle est raccordée fluidiquement une extrémité amont (6a) d'un conduit flexible (6),
dans lequel :
- la paroi de toit (4) comprend une ouverture supérieure (7) en communication avec au moins une partie dudit espace interne (3), au moins une partie intermédiaire (6b) dudit conduit flexible (6) venant se loger dans ladite au moins une partie de l'espace interne (3) au travers de l'ouverture supérieure (7), et
- un compartiment de rangement (11) est agencé dans l'espace interne (3) de la carcasse (2) en-dessous de l'ouverture supérieure (7), ladite ouverture supérieure (7) débouchant dans ledit compartiment de rangement (11) de manière à ce qu'au moins une partie intermédiaire (6b) du conduit flexible (6) vienne se loger dans ledit compartiment de rangement (11),
**caractérisé en ce que** le compartiment de rangement (11) comprend un sac à paroi périphérique souple (11a).

2. Chariot (1) selon la revendication 1, **caractérisé en ce que** le système de distribution (5) de gaz comprend une extrémité de raccordement (5a) située à l'extérieur de la carcasse (2).

3. Chariot (1) selon la revendication 1, **caractérisé en ce que** le sac à paroi périphérique souple (11a) formant le compartiment de rangement (11) comprend des moyens de fixation permettant de le fixer à la carcasse (2).

4. Chariot (1) selon la revendication 3, **caractérisé en ce que** le sac à paroi périphérique souple (11a) comprend des moyens de fixation permettant de le fixer à la paroi de toit (4) de la carcasse (2).

5. Chariot (1) selon la revendication 1, **caractérisé en ce que** le conduit flexible (6) comprend une extrémité amont (6a), une extrémité aval (6c) à raccorder fluidiquement à une interface respiratoire (8) et une partie intermédiaire (6b) située entre lesdites extrémité amont (6a) et aval (6c).

6. Chariot (1) selon les revendications 1 et 5, **caractérisé en ce que** l'extrémité amont (6a) et l'extrémité aval (6c) sont situées hors de la carcasse (2) lorsque la partie intermédiaire (6b) du conduit flexible (6) est logée dans le compartiment de rangement (11).

7. Chariot (1) selon la revendication 1, **caractérisé en ce qu'**un dispositif de guidage (12) est agencé au niveau de l'ouverture supérieure (7) pour guider le conduit flexible (6) lorsqu'il est introduit ou extrait du compartiment de rangement (11) au travers de l'ouverture supérieure (7).

8. Chariot (1) selon la revendication 7, **caractérisé en ce que** le dispositif de guidage (12) a une forme générale de diabolo comprenant un passage central (12a) logeant la partie intermédiaire (6b) du conduit flexible (6).

9. Chariot (1) selon la revendication 8, **caractérisé en ce que** le dispositif de guidage (12) a une forme générale de diabolo comprenant en outre des parois supérieure et inférieure (121, 122) formant des flasques parallèles séparées par une gorge annulaire (120), lesdites deux parois supérieure et inférieure (121, 122) venant prendre en sandwich les surfaces supérieure et inférieure de la paroi de toit (4), au niveau du rebord périphérique (7a) de l'ouverture supérieure (7), et le rebord périphérique (7a) de l'ouverture supérieure (7) venant se loger dans la gorge (120).

10. Chariot (1) selon l'une des revendications 1, 5 ou 6, **caractérisé en ce qu'**un contrepoids coulissant (13) est porté par la partie intermédiaire (6b) du conduit flexible (6).

11. Chariot (1) selon la revendication 1, **caractérisé en ce que** le sac à paroi périphérique souple (11a) comprend des moyens de fermeture permettant de fermer le sac, après insertion totale du conduit flexible (6) dans le volume interne dudit sac, de préférence conduit flexible (6) et d'une interface respiratoire (8) fixée audit conduit flexible (6).

12. Chariot (1) selon la revendication 1, **caractérisé en ce que** le sac à paroi périphérique souple (11a) est formé de plastique.

13. Utilisation d'un chariot (1) selon l'une des revendications précédentes pour transporter au moins une bouteille de gaz (20), de préférence une ou des bouteilles de gaz contenant un gaz ou mélange gazeux choisi parmi l'oxygène, l'air et un mélange N₂O/O₂.
